# EUROPEAN PATENT APPLICATION

(11) **EP 2 591 797 A1**
(43) Date of publication of application: **15.05.2013**
(21) Application number: 11803163.2
(22) Date of filing: 11.07.2011
(51) Int. Cl.: A61K 38/53, A61K 38/16, A61P 1/00, A61P 11/00, A61P 13/12, A61P 15/00, A61P 17/00, A61P 19/00, A61P 31/00, A61P 33/00, A61P 35/00, A61P 37/00

(54) **REGULATORY FACTOR OF FOXP3 AND REGULATORY T CELLS AND USE THEREOF**

(30) Priority: 09.07.2010 CN 201010222995
(71) Applicant: Institut Pasteur Of Shanghai, Cas, Shanghai 200025 (CN)
(72) Inventor: LI, Bin, Shanghai 200025 (CN); LIN, Fang, Shanghai 200025 (CN); CHEN, Zuojia, Shanghai 200025 (CN); LI, Zhiyuan, Shanghai 200025 (CN); PAN, Fan, Shanghai 200025 (CN)
(74) Representative: Giovannini, Francesca
(86) International application number: PCT/CN2011/077032
(87) International publication number: WO 2012/003810

(57) **Abstract**

The present invention relates to regulatory factors for FOXP3 and regulatory T cells and application thereof. Specifically, the present invention relates to the use of an ubiquitination pathway-related factor, its agonist or antagonist in the preparation of a composition for regulating FOXP3, IL-2, and/or IFN-γ activity, in which the ubiquitination pathway-related factor is selected from: Toll-like receptor, ubiquitin ligase, pro-inflammatory cytokine family receptor, and/or its coding sequence. The new type of regulatory factors of the present invention can regulate regulatory T cells and immune system by regulating FOXP3, IL-2, and/or IFN-γ activity. The regulatory factors and their derivatives of the present invention can also be used as immunoadjuvant for treating or preventing major diseases (such as, infectious diseases and tumor, etc).

## Description

### BACKGROUND OF INVENTION

### Field of the Invention

The present invention relates to the fields of molecular biology and biomedicine. More specifically, the invention relates to use of the ubiquitination pathway-related factors, the agonist or antagonist in the regulation of FOXP3 activity, IL-2 activity and/or IFN-γ activity. The invention particularly relates to regulation of the negative regulatory factor of FOXP3 activity, and by down regulating FOXP3 activity to modulate the immune system, resulting in the use for treatment or prevention of diseases or symptoms associated with excessive FOXP3 activity and as an immune adjuvant.

### Background Art

The Fox transcription factor family is a superfamily of transcription factors having the characteristics of fork head helix (forkhead/winged helix, FKH) with different functions. Members of this family play different roles in various processes during cell development (refs 1 and 2).

FOXP3 is a specific transcription factor of the regulatory T cells (Treg, regulatory T cells), plays an important regulatory role in Treg development and function, and has the function of down regulating immune response. FOXP3-mediated Treg immune regulatory function is through forming protein complexes of FOXP3 and several transcriptional co-regulator proteins (such as transcription factors, co-repressors, co-activators, histone proteins, and chromatin remodeling factors) to dynamically regulate specific gene transcription (refs 3 and 4). Under different stimulus conditions, different states of FOXP3 protein complexes and their transcriptional activity have different effects on Treg and immune system modulation (refs 3 and 4).

FOXP3+ regulatory T cells (FOXP3+Tregs) belongs to a class of T cell subsets of T lymphocytes expressing CD4, CD25, and transcription factor FOXP3, and their normal function is essential for dynamic regulation of human immune homeostasis. De-regulation of T cell development and function is closely associated with physiological and pathological processes of a variety of major immune associated diseases, including autoimmune diseases, inflammation, acute and chronic infectious diseases, tumor immune tolerance, transplant rejection, and allergic diseases (refs 1 and 2).

Although research in this field has progressed rapidly in recent years, but many significant and fundamental questions still remain to be answered, for example, how Treg cells regulate other immune cell activity at the cellular level, and what mechanisms at the molecular level FOXP3 uses to acquire the immunosuppressive activity of regulatory T cells.

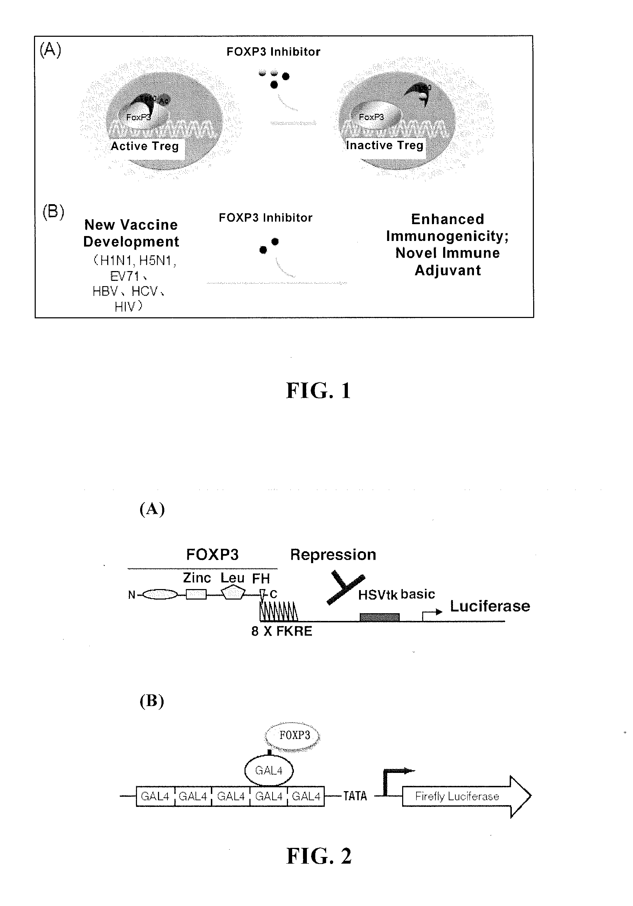

Accumulating experimental evidence shows, gene expression levels and continuing state of FOXP3 are crucial for the natural development and function of regulatory T cells. In addition, FOXP3 interacts with multiple transcription factors and histone acetyltransferase/deacetylase complexes having enzymatic activity and that is required for inhibiting transcription activation of pro-inflammatory cytokines in T cells. And, post-translational modification of FOXP3, transcription complex assembly, and its modification enzyme activity are dynamically regulated by T cell receptor and inflammatory cytokine receptor signaling. Previous studies found that FOXP3 is a protein with acetylated lysines, and the proline-rich N-terminal of FOXP3 can directly recruit histone acetyltransferase TIP60 (Tat interaction protein, 60 kDa), to mediate the transcriptional repression activity of FOXP3 (see ref. 6 and FIG. 1).

IL-2 is a key cytokine, and is usually secreted from lectin- or antigen-activated T lymphocytes. It can induce proliferation of cytotoxic T cells, natural killer cells, and lymphokine-activated killer cells, and can enhance the killing activity, can promote the secretion of antibodies and interferons of lymphocytes, having the functions of anti-virus, anti-tumor, enhancing immunity, etc. Recombinant human IL-2 has corresponding effects on tumor, inflammation, acute infectious diseases, or chronic infectious disease.

IFN-γ is a dimerized soluble cytokine, and is usually produced by natural killer cells, natural killer T cells, and cytotoxic T cells. IFN-γ is critical for innate and adaptive immune system against viral and intracellular bacterial infections. It can directly inhibit viral replication *in vivo,* having antiviral, immunoregulatory, and anti-tumor properties. IFN-γ are activated through its interaction with a heterodimeric receptor to regulate JAK-STAT pathways, activate antigen-presenting cells, and induce Type I T helper cells (Thl cells) differentiation through up-regulation of transcription factor T-bet. It can induce antiviral protein synthesis, treat virus infectious diseases; can down-regulate the mRNA levels of hepatic type I and III collagen, and prevent and slow down the progression of liver fibrosis, indicating its effectiveness in chronic active hepatitis and liver cirrhosis.

Therefore, the studies of the biochemical activity, physiological functions and the molecular mechanisms of regulatory T cells, FOXP3, IL-2 and IFN-γ are important for the regulation of the activity of Treg cells in immune homeostasis and immunotherapy based on regulatory T cells. This provides new research issues and challenges in translating basic immunology research into clinical studies, and in understanding major human infectious diseases.

### SUMMARY OF INVENTION

One of the main objects of the present invention is to provide uses of ubiquitination pathway-related factors and their agonists or antagonists in the regulation of FOXP3 activity, IL-2 activity and/or IFN-γ activity. Another object of the present invention is to provide uses of these regulatory factors in the regulation of FOXP3 activity, IL-2 activity and/or IFN-γ activity to regulate the immune system. Another object of the present invention is to provide methods of using the regulatory factors of the present invention in the treatment of regulatory T cells associated diseases, and application thereof.

In the first aspect of the present invention, it provides the uses of ubiquitination pathway-related factors, their agonists or antagonists in preparation of compositions for the regulation of FOXP3 activity, IL-2 activity and/or IFN-γ activity, wherein the ubiquitination pathway-related factors are selected from: Toll like receptors, ubiquitin ligase, pro-inflammatory cytokine family receptors, and/or their coding sequences.

In one embodiment of the present invention, the regulation of FOXP3 activity, IL-2 activity, and/or IFN-γ activity is a positive or negative regulation, wherein the ubiquitination pathway-related factors or their agonists are used for the negative regulation of FOXP3 activity and for the positive regulation of IL-2 activity and/or IFN-γ activity, wherein the antagonists of ubiquitination pathway-related factors are for the positive regulation of FOXP3 activity and for the negative regulation of IL-2 activity and/or IFN-γ activity.

In a preferred embodiment, the agonist is IL-6.

In another preferred embodiment, the antagonist is MG-132.

In another embodiment of the present invention, a receptor of the pro-inflammatory cytokine family is selected from: IL-6R, TGF-β receptor, IL-2 receptor, TNF-α receptor, or GITR.

In a preferred embodiment, the use is using a ubiquitination pathway-related factor in the preparation of a composition for the negative regulation of FOXP3 activity, wherein the ubiquitination pathway-related factor is selected from: a Toll like receptor, a ubiquitin ligase, and/or their coding sequences.

In another embodiment of the present invention, the ubiquitin ligase is one or more selected from the following: STUB1, TRAF6, Smurf1, Smurf2, TRAF2, TRAF3, TRAF4, p300, RNF31, or RBCK1

In a preferred embodiment, the ubiquitin ligase is preferably STUB1.

In another preferred embodiment, the ubiquitin ligase is a complex, which also has a molecular chaperone activity.

In another preferred embodiment, the ubiquitin ligase is a downstream target of Toll like receptor signaling pathway, preferably TRAF6.

In another embodiment of the present invention, the Toll like receptor is one or more selected from the following: TLR1, TLR2, TLR3, TLR4, TLR5, TLR7, TLR8, or TLR9.

In a preferred embodiment, the Toll like receptor is selected from: TLR3, TLR4, TLR7, TLR8, or TLR9.

In another preferred embodiment, the downstream kinase IRAK1 of Toll like receptor signaling pathway can induce FOXP3 ubiquitination.

In another embodiment of the present invention, the composition for the regulation of FOXP3 activity, IL-2 activity, and/or IFN-γ activity is used for the treatment and/or prevention of diseases or disorders associated with de-regulation of FOXP3, IL-2, and/or IFN-γ activity.

In a preferred embodiment of the invention, the de-regulated activity is an excessively high activity or an excessively low activity.

In another embodiment of the present invention, the diseases or symptoms associated with the de-regulated FOXP3, IL-2, and/or IFN-γ activity are selected from: tumor, inflammation, acute infectious diseases, or chronic infectious diseases.

In another embodiment of the present invention, the tumor is selected from: prostate cancer, breast cancer, liver cancer, glioma, colorectal cancer, cervical cancer, non small cell lung cancer, lung cancer, pancreatic cancer, gastric cancer, bladder cancer, skin cancer, striated muscle cancer, tongue squamous cell carcinoma, nasopharyngeal carcinoma, ovarian cancer, placental villous cancer, neuroglioma, lymphoma, leukemia, rectal adenocarcinoma, or melanoma.

In another embodiment of the present invention, the inflammatory response is selected from: allergic inflammation, folliculitis, tonsillitis, pneumonia, hepatitis, nephritis, acne, asthma, autoimmune diseases, chronic inflammation, chronic prostatitis, glomerulonephritis, hypersensitivity, inflammatory bowel disease, pelvic inflammatory disease, reperfusion injury, rheumatoid arthritis, transplant rejection, vasculitis, or interstitial cystitis.

In another embodiment of the present invention, the infectious disease is selected from: plague, cholera, SARS, AIDS, viral hepatitis, polio, human infection of highly pathogenic avian influenza, measles, epidemic hemorrhagic fever, rabies, epidemic type B encephalitis, hand-foot and mouth disease, dengue fever, anthrax, bacillary and amebic dysentery, tuberculosis, typhoid and paratyphoid fever, epidemic cerebrospinal meningitis, pertussis, diphtheria, tetanus, scarlet fever, brucellosis, gonorrhea, syphilis, leptospirosis, schistosomiasis, malaria, influenza, mumps, rubella, acute hemorrhagic conjunctivitis, leprosy, epidemic and endemic typhus, visceral leishmaniasis, echinococcosis, filariasis, and infectious diarrheal diseases other than cholera, bacillary and amebic dysentery, typhoid, or paratyphoid fever, or a fungal infection.

In another embodiment of the present invention, the composition for the regulation of FOXP3, IL-2 activity, and/or IFN-γ activity is further used as a vaccine adjuvant or a vaccine.

In another embodiment of the present invention, the vaccine is selected from: vaccines for the treatment or prevention of viral infection, bacterial infection, inflammation, parasitic infection, or tumor, such as hand-foot-and-mouth disease vaccine.

In a preferred embodiment, the vaccine is selected from: tetanus vaccine, diphtheria, tetanus, pertussis vaccine, influenza vaccine, yellow fever vaccine, poliomyelitis vaccine, tuberculosis vaccine, measles vaccine, rubella vaccine, type A influenza meningitis vaccine, rabies vaccine, hepatitis B vaccine, measles/mumps/rubella vaccine, haemophilus influenza type B vaccine, typhoid, hepatitis A vaccine, cervical cancer vaccine, pneumonia vaccine, varicella vaccine, epidemic cerebrospinal meningitis vaccine, Japanese encephalitis vaccine, dysentery vaccine, cholera vaccine, AIDS vaccine, hepatitis C vaccine, hepatitis E vaccine, hand-foot-and-mouth disease vaccine, bronchitis vaccine, A-group epidemic cerebrospinal meningitis vaccine, or human avian influenza vaccine.

In another preferred embodiment, the vaccine m a DNA vaccine, a recombinant vector vaccine, a vaccine based on an inactivated or attenuated pathogen, a subunit vaccine, or a cancer cell vaccine.

In another embodiment of the present invention, the composition is a pharmaceutical composition, a health product composition, or a vaccine composition.

In a preferred embodiment, a regulatory factor content in the composition is 0.05-99.5 weight%, preferably 0.1-95 weight%, more preferably 1-90 weight %, more preferably 5-80 weight %.

In another preferred embodiment, the composition further comprises a molecular chaperone required for ubiquitination, the molecular chaperone is preferably: HSP70, HSC70, or HSP90.

In another preferred embodiment, the composition further comprises a pharmaceutically, health science, or immunologically acceptable carrier.

In another preferred embodiment, the composition is in a dosage form of an injection, a tablet, granules, powders, or a capsule.

In a second aspect of the invention, it provides a composition for the regulation (such as negative regulation) of FOXP3 activity, IL-2 activity, and/or IFN-γ activity, which comprises:
(a) one or more selected from the group consisting of ubiquitination pathway-related factors: ubiquitin ligases, Toll like receptors, pro-inflammatory cytokine family receptors and/or their coding sequences; and/or the ubiquitination pathway-related factor agonists or antagonists;
(b) a pharmaceutically, immunologically, or health science acceptable carrier.

In a preferred embodiment, the composition is used for the treatment or prevention of a disease or symptom associated with de-regulation of FOXP3, IL-2, and/or IFN-γ activity (i.e., excessively high activity or excessively low activity) or used as a vaccine adjuvant.

In another preferred embodiment, the composition is further used in the preparation of a vaccine composition; the vaccine composition further comprises an active substance having an immunogenic activity.

In a preferred embodiment, the composition further comprises one or more other active substances capable of regulating FOXP3, IL-2, and/or IFN-γ, preferably TIP60.

In one embodiment of the invention, the ubiquitin ligase is one or more selected from the following: STUB1, TRAF6, Smurf1, Smurf2, TRAF2, TRAF3, TRAF4, p300, RNF31, or RBCK1.

In a preferred embodiment, the ubiquitin ligase is preferably STUB1.

In another preferred embodiment, the ubiquitin ligase is a complex, which also has a molecular chaperone activity.

In another preferred embodiment, the ubiquitin ligase is a downstream activating protein of Toll like receptor signaling pathway, and preferably TRAF6.

In another embodiment of the present invention, the Toll like receptor is one or more selected from the following: TLR1, TLR2, TLR3, TLR4, TLR5, TLR7, TLR8, or TLR9.

In a preferred embodiment, the Toll like receptor is selected from: TLR3,TLR4, or TLR9.

In another preferred embodiment, the downstream kinase IRAK1 of the Toll like receptor signaling pathway can induce FOXP3 ubiquitination.

In another embodiment of the present invention, the composition for the regulation of FOXP3 activity, IL-2 activity, and/or IFN-γ activity is used for the treatment and/or prevention of a disease or symptom associated with de-regulation of FOXP3, IL-2, and/or IFN-γ activity.

In another embodiment of the present invention, the disease or symptom associated with de-regulated activity is selected from: tumor, inflammation, acute infectious diseases, or chronic infectious diseases. The tumor, inflammation, acute infectious diseases, or chronic infectious diseases may be those described above.

In another preferred embodiment, the vaccine is a DNA vaccine, a recombinant vector vaccine, a vaccine based on an inactivated or attenuated pathogen, a subunit vaccine, or a cancer cell vaccine.

In another embodiment of the present invention, the composition is a pharmaceutical composition, a health product composition, or a vaccine composition.

In other aspects of the invention, it provides methods for regulating FOXP3 activity, IL-2 activity, and/or IFN-γ activity. A method comprises administering one or more regulatory factors or agonists thereof or antagonists thereof selected from the following: ubiquitin ligase, Toll like receptor, pro-inflammatory cytokine family receptors and/or their coding sequences.

In a preferred embodiment, the ubiquitin ligase is one or more selected from the following: STUB1, TRAF6, Smurf1, Smurf2, TRAF2, TRAF3, TRAF4, p300, RNF31, or RUCK1, preferably STUB1. In another preferred embodiment, the Toll like receptor is one or more selected from the following: TLR1 TLR2, TLR3, TLR9, TLR4, TLR5, TLR7, or TLR8, preferably TLR3, TLR4 or TLR9.

In another preferred embodiment, the pro-inflammatory cytokine family receptor is one or more selected from the following: IL-6R, TGF-β receptor, IL-2 receptor, TNF-α receptor, or GITR.

In another preferred embodiment, the method is further used for the treatment or prevention of a disease or symptom associated with de-regulation of FOXP3, IL-2, and/or IFN-γ activity.

In another preferred embodiment, the method is further used for enhancing vaccine immunogenicity.

Other aspects of the invention, as a result of the present disclosure, would be apparent to one skilled in the art.

### BRIEF DESCRIPTION OF DRAWINGS

FIG. 1: Application model of FOXP3 inhibitors known in the prior art. FIG. 1 (A): FOXP3 inhibitors can inhibit protein (such as Tip60) binding of FOXP3 functional complex, thereby inhibiting Treg function; FIG. 1 (B): FOXP3 inhibitors can be used as a vaccine adjuvant, enhancing vaccine immunogenicity.

FIG. 2: FOXP3 transcriptional activity model. Use cellular luciferase system to report the effects of inhibitors on FOXP3 transcription activity by detecting the luciferase activity treated by different inhibitors. FIG. 2 (A): A model of using 8XFK Luciferase Reporter System to detect FOXP3 transcription activity; FIG. 2 (B): A model of using 5XGal Luciferase Report System to detect FOXP3 transcription activity.

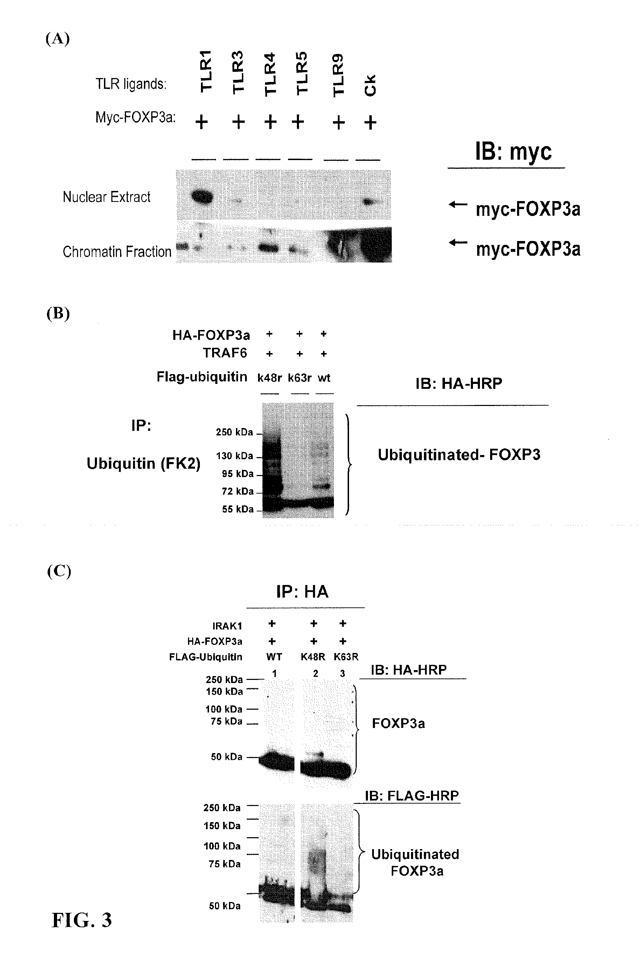

FIG. 3: Effects of TLR signaling and the downstream activating protein on FOXP3. FIG. 3 (A): Effect of TLR signaling on FOXP3 expression. FIG. 3 (B): Effect of TLR signaling downstream activating protein TRAF6 on FOXP3 ubiquitination. FIG. 3 (C): Effect of TLR signaling downstream activating protein IRAK1 on FOXP3 ubiquitination.

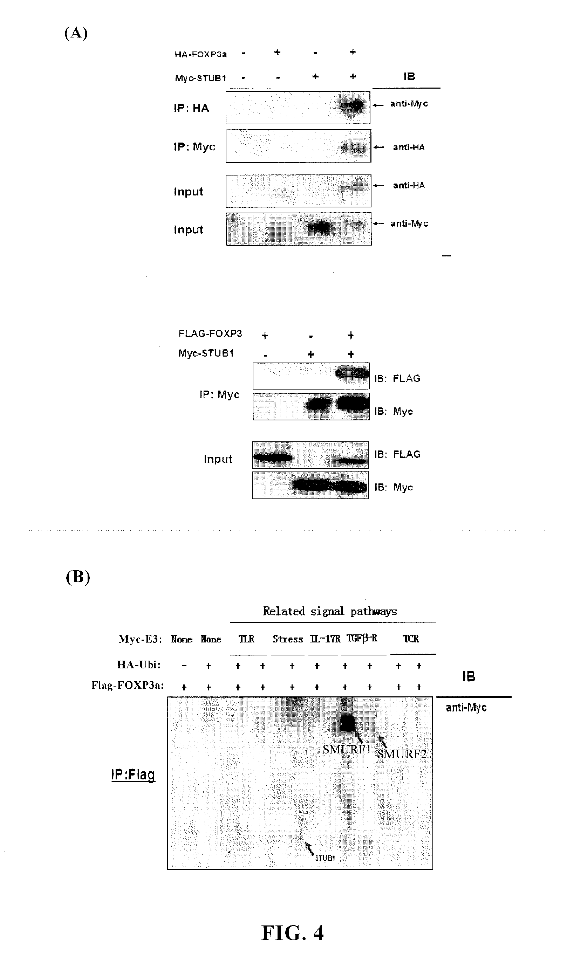

FIG. 4: FOXP3 binding to STUB1 and other E3. FIG. 4 (A): left panel: 293T cells were co-transfected with HA-FOXP3a and Myc-STUB1, co-immunoprecipitated using anti-HA and anti-Myc antibodies, and detection by immunoblotting; right panel: in a reversed confirmation experiments, 293T cells were co-transfected with Flag-FOXP3 and Myc-STUB1, co-immunoprecipitated using anti-Flag and anti-Myc antibodies, and detection by immunoblotting. FIG. 4 (B): Detection of other immune signaling pathways by co-transfecting E3 and Flag-FOXP3a, HA-Ubi into 293T cells. Cell lysate was immunoprecipitated using anti-Flag antibody, and detected using anti-Myc antibody.

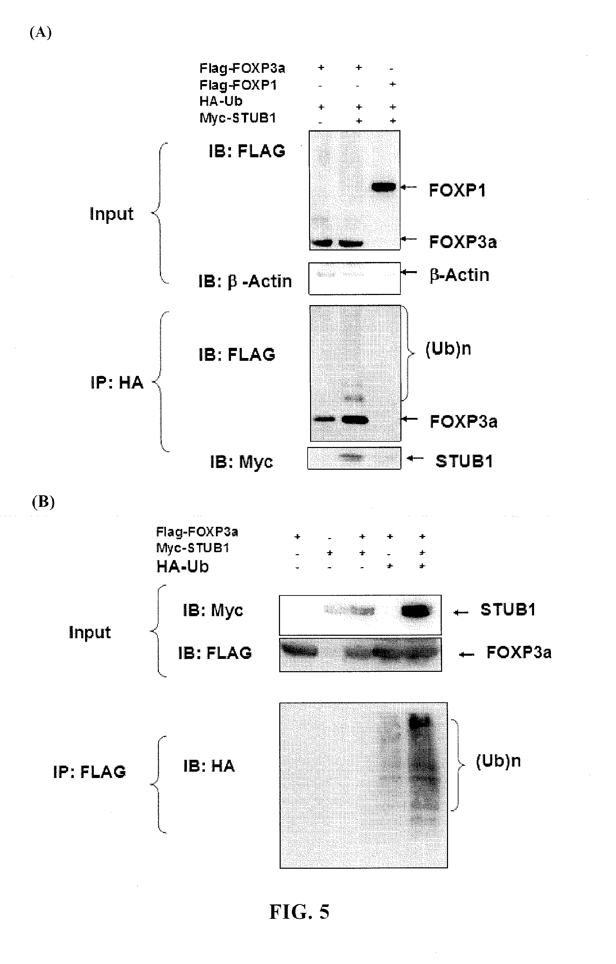

FIG. 5: STUB1 can cause FOXP3 ubiquitination. FIG. 5 (A): 293T cells were co-transfected with Myc-STUB1, HA-Ub and Flag-FOXP3a or Flag-FOXP1. Cell lysates were immunoprecipitated using anti-HA antibody, and ubiquitinated proteins were detected by immunoblotting; FIG. 5 (B): Jurkat T cells were co-transfected with Myc-STUB1, HA-Ub and Flag-FOXP3a, cell lysates were immunoprecipitated using anti-HA antibody and detected with immunoblotting.

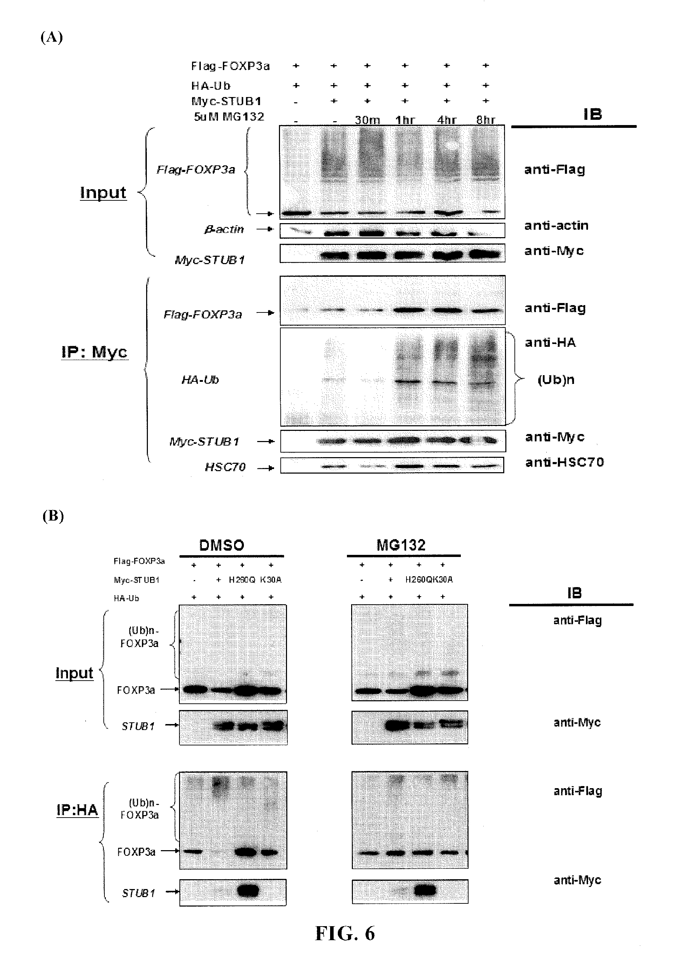

FIG. 6: STUB1 promotes FOXP3 degradation by ubiquitination. FIG. 6 (A): 293T cells were co-transfected with Flag-FOXP3a, HA-Ub and Myc-STUB1. Before the cells were harvested, treated the cells with 5 µM MG132 at different time points. Lysates were immunoprecipitated using anti-Myc antibody and detected with immunoblotting. FIG. 6 (B): 293T cells were co-transfected with Flag-FOXP3a, HA-Ub and Myc-STUB1 and their mutants. After cells were treated with 5 µM MG132 for 4 hours, the cells were immunoprecipitated using anti-HA antibody and detected with immunoblotting.

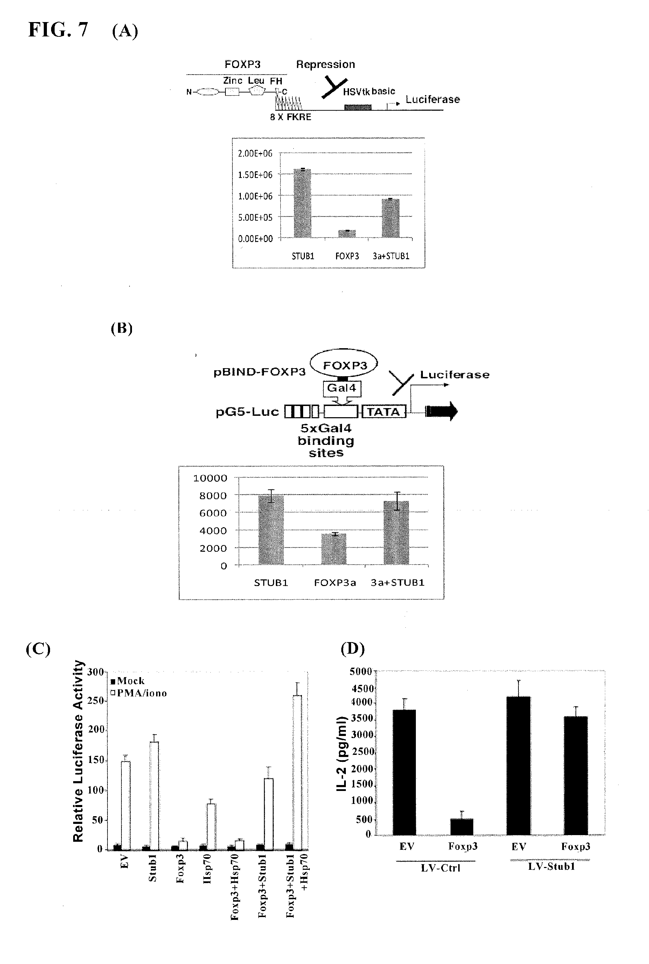

FIG. 7: STUB1 inhibits FOXP3 transcription activity. FIG. 7 (A): 8XFK Luciferase Report System was used to detect FOXP3 activity. 293T cells were co-transfected with FOXP3, STUB1, 8XFK Luciferase reporter gene and MSV-P-Gal controls. After using β-Gal to normalize the luciferase activity, results were shown as mean value ± standard deviation using three independent experiments; FIG. 7 (B): 293T cells were co-transfected with pBind-FOXP3, STUB1, and pGal5-luciferase reporter gene and MSV-P-Gal controls using pBind system. After using β-Gal to normalize the luciferase activity, results obtained from three independent experiments were shown as mean value ± standard deviation. FIG. 7 (C): Effects of overexpression of HSP70 and STUB1 on the expression levels of a luciferase reporter gene driven by FOXP3-mediated IL-2 promoter. FIG. 7 (D): Using lentivirus-mediated transfection methods to express full-length FOXP3 protein and STUB1 in primary CD4+T cells to study whether STUB1 can alleviate the inhibitory effect of FOXP3 on IL-2 expression.

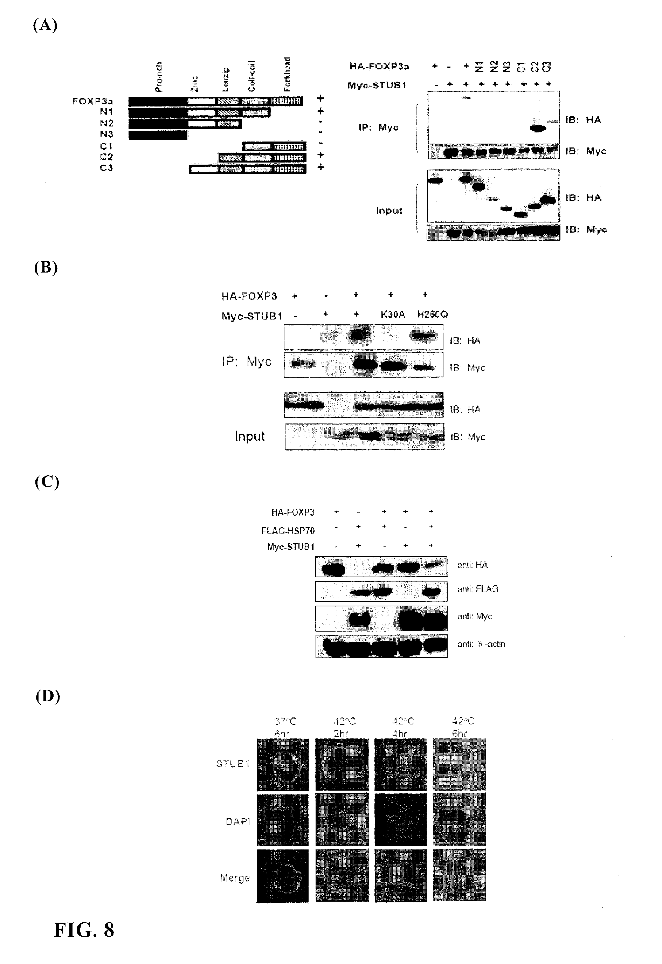

FIG. 8: Identification of FOXP3 critical domain interacting with STUB1 (FIG. 8 (A)), effects of molecular binding partner on the interaction (FIG. 8 (B) and 8 (C)), and the possible mechanism (FIG. 8 (D)).

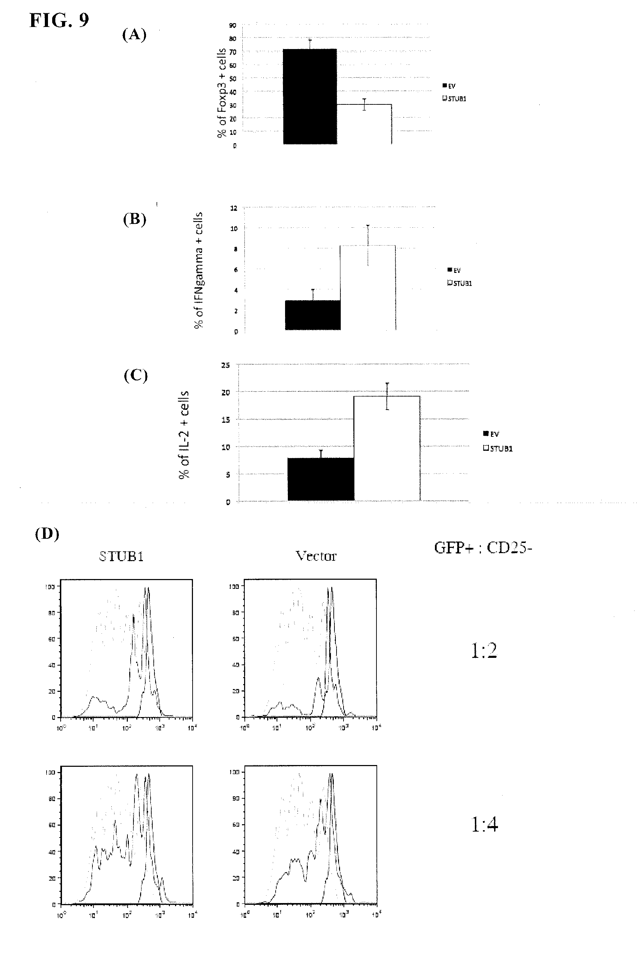

FIG. 9: Study of *in vitro* negative regulation of FOXP3 protein expression by STUB1 and the function of primary mouse Treg cells. STUB1 overexpression reduces the expression of intracellular FOXP3 (FIG. 9 (A)), while increasing the expression the inflammatory factor IL-2 (FIG. 9 (B)) and IFN-γ (FIG. 9 (C)); experimental results of cell proliferation (FIG. 9 (D)), curve 1 represents the addition of only CD25⁻, curve 2 represents GFP⁺CD25⁻, and curve 3 represents non-activated CD25⁻.

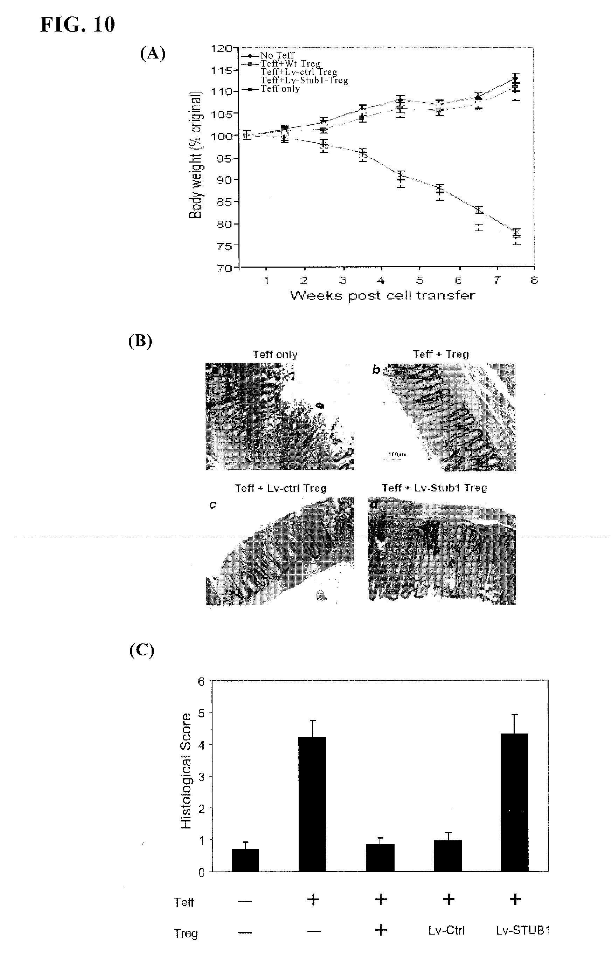

FIG. 10: STUB1 overexpression in Treg cells can weaken immune suppression of Treg cells in an experimental colitis mouse model.

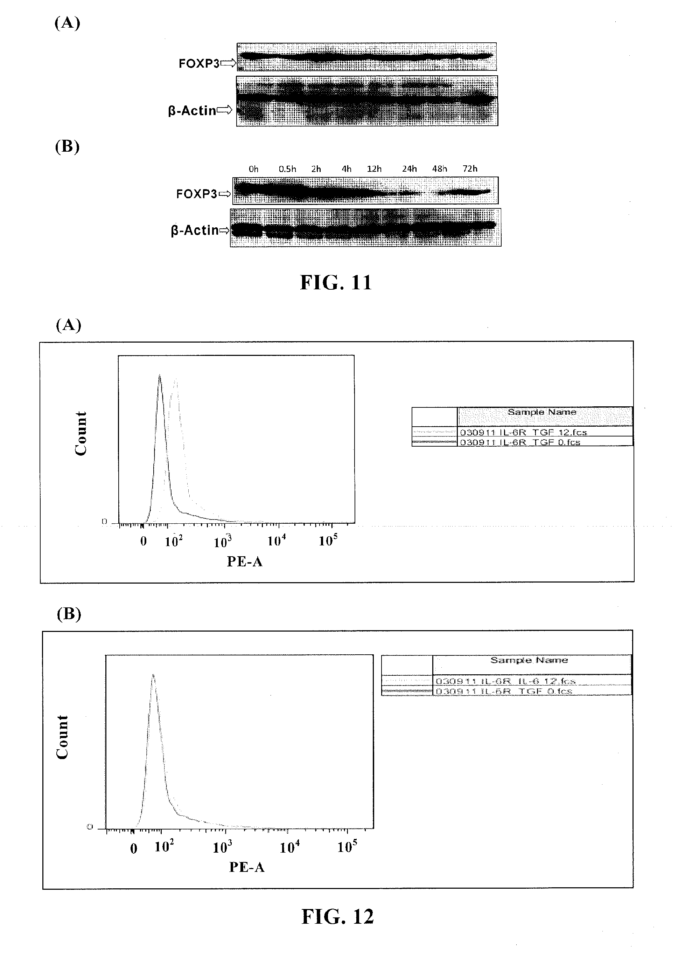

FIG. 11: Verification, using Western blotting, of the synergistic effect of IL-6 and TGF-beta in promoting FOXP3 degradation. FIG. 11 (A): FOXP3 degradation by IL-6 stimulation; FIG. 11 (B): FOXP3 degradation by IL-6/TGF-β stimulation.

FIG. 12: Verification, using FACS, of the effects of TGF-β and IL-6 stimulations on the expression levels of IL-6 receptor. FIG. 12 (A): the expression levels of IL-6 receptor under TGF-β stimulation; FIG. 12 (B): the expression levels of IL-6 receptor under TGF-β stimulation.

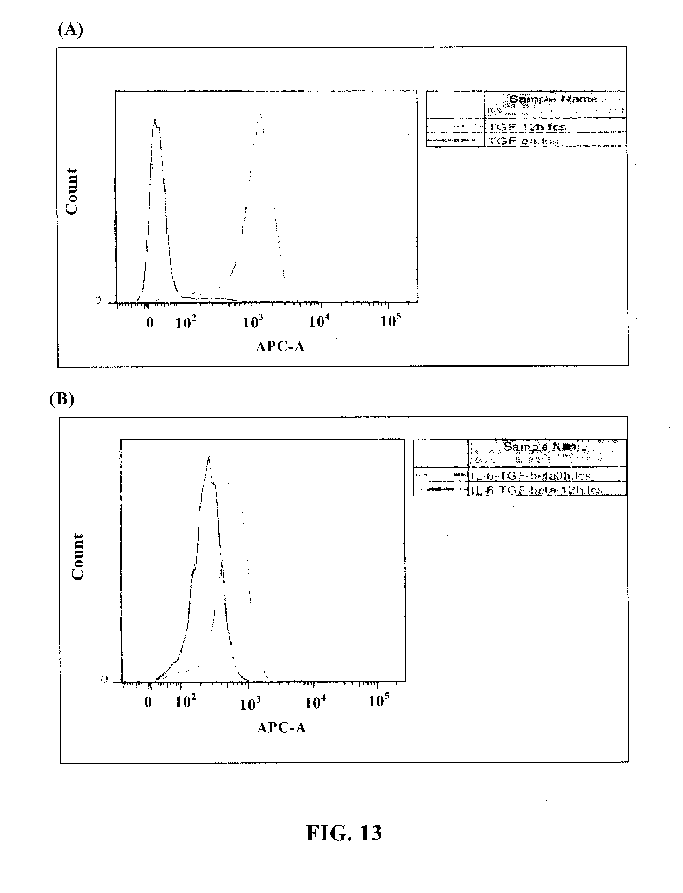

FIG. 13: Verification, using FACS, of the effects of TGF-β and IL-6/TGF-β stimulations on the expression levels of FOXP3. FIG. 13 (A): the expression levels FOXP3 under TGF-β stimulation; FIG. 13 (B): the expression levels FOXP3 under IL-6/TGF-β stimulation.

In the above figures, IP refers to immunoprecipitation, and IB refers to immunoblotting.

### DETAILED DESCRIPTION

The present inventors believe that FOXP3 can be regulated temporally and spatially, including post-transcriptional modification, translation or modification and protein interactions, etc. One type of factors, which can function as FOXP3 negative regulatory factors, include enzymes, small molecular compounds, etc. These factors can down regulate FOXP3 activity under different signaling stimulations, thereby regulating the entire immune system. Through long-term and thorough research, the present inventors discovered that the ubiquitination pathway-related factors can down regulate FOXP3 activity. The ubiquitination pathway-related factors are FOXP3 negative regulatory factors - Toll like receptor, ubiquitin ligase, pro-inflammatory cytokine family receptors and/or their coding sequences.

Specifically, the present inventors discovered: under the innate immune signaling, such as different TLR signaling (such as TLR3 ,TLR4 and TLR9), can cause FOXP3 protein degradation, indicating the presence of negative regulators of FOXP3 protein. In addition, the present inventors, through further study, also discovered another important post-translational modification - ubiquitination, present in FOXP3 protein. After further research, the present inventors first discovered a close relationship between TLR signaling pathway and ubiquitination: TLR signaling downstream ubiquitin ligases, such as TRAF6, or protein kinases, such as IRAK1, and multiple cell type recognition receptors (such as, IL-6 etc.), which can promote FOXP3 protein ubiquitination.

Ubiquitination generally occurs in many physiological regulation, leading to changes in activity and function of the modified protein substrates. Protein degradation by ubiquitination is also a major pathway for degrading proteins, for example, K48- ubiquitination causes protein degradation through proteasome. There are many important ubiquitin ligases (E3) in immune signaling pathways, such as TRAF6 in TLR pathways, and Smurf1 under TGF-β signaling.

The present inventors tested each of these E3 and found that E3 can induce FOXP3 ubiquitination. For example, the stress signaling-activated E3, STUB1 (STIP1 homology and U-box containing protein 1)/CHIP (C terminus of Hsc70-interacting protein), can clearly lead to FOXP3 degradation through ubiquitination.

STUB1 can bind to FOXP3 protein mediated by molecular binding partners. of the STUB1 with K30A mutation cannot bind to molecular binding partner proteins, and cannot bind to FOXP3. Whereas, STUB1 with H260 mutation loses E3 activity, still can bind to FOXP3, but cannot promote ubiquitination. The present inventors, in FOXP3 transcription activity model, found that STUB1 overexpression can significantly decrease the FOXP3 transcriptional activity. Thus, on the posttranslational modification level, STUB1 acts as a FOXP3 negative regulatory factor, inducing FOXP3 degradation through ubiquitination mediated by molecular binding partners.

Moreover, *in vivo* and *in vitro* experiments have further confirmed that STUB1 can negatively regulate FOXP3 protein expression and the function of primary mouse Treg cells.

In addition, the present inventors also found STUB1 overexpression also can increase the expression of inflammatory factors IL-2 and IFN-γ, and can reverse the inhibitory effect of FOXP3 on IL-2 expression. Thus, it confirms that the ubiquitination pathway-related factors and their agonists or antagonists can also be used to regulate IL-2 and/or IFN-γ activity.

Based on the above research, the present inventors, in the present invention, provides and discloses, for the first time, a use of ubiquitination pathway-related factors - - ubiquitin ligases, Toll like receptors, pro-inflammatory cytokine family receptors and/or their coding sequences and their agonists or antagonists as regulatory factors for FOXP3 activity, IL-2 activity, and/or IFN-γ activity. The confirmation and application of these regulatory factors, through regulating FOXP3, IL-2, and/or IFN-γ activity to regulate regulatory T cells, provide an approach for treating and/or preventing diseases or symptoms (e.g. tumor) associated with de-regulation of FOXP3, IL-2, and/or IFN-γ activity. They also provide new methods for enhancing immunogenicity of viral infection using these regulatory factors as vaccine adjuvant. On this basis, the present inventors have introduced of the present invention to practice.

### Ubiquitination pathway-related factors, their agonists or antagonists

As used herein, the term "regulators" or "regulatory factors of activity" refers to ubiquitination pathway-related factors or their agonists or antagonists. They can regulate the FOXP3, IL-2, and/or IFN-γ activity through affecting ubiquitination pathway, thus further can be used in the prevention and/or treatment of diseases or symptoms associated with de-regulation of FOXP3, IL-2, and/or IFN-γ activity.

As used herein, the term "ubiquitination pathway-related factors" refers to factors that can decrease FOXP3 activity through affecting ubiquitination pathway. In the present invention, the ubiquitination pathway-related factors include: ubiquitin ligases, pattern recognition receptors (such as Toll like receptors), pro-inflammatory cytokine family receptors, and/or their coding sequences.

As used herein, the term "Toll like receptors" or "TLR" are interchangeable, refers to the specific type I-transmembrane receptors and pathogen type recognition receptors in the natural immune system, playing an important role in acute inflammatory reaction, cell signal transduction, and cell apoptosis.

So far, 10 human TLR functional family members (TLR1-TLR10) have been identified. Based on the conservation of chromosomal distribution, gene structures, and the amino acid sequences, human TLRs receptors can be divided into 5 subtypes, i.e., TLR2, TLR3, TLR4, TLR5, and TLR9 subgroups. TLR2 subgroup includes TLR1, TLR2, TLR6, and TLR10. TLR9 subgroup includes TLR7 and TLR9. TLR3, TLR4 and TLR5 each form a subgroup.

The present invention discloses, for the first time, the negative regulatory effect of TLR on FOXP3. TLR used in the preferred embodiments of the present invention includes (but not limited to): TLR1, TLR2, TLR3, TLR4, TLR5, TLR7, or TLR8, TLR9, preferably TLR of TLR2, TLR3, TLR4, and TLR9 subgroups, more preferably TLR3, TLR4,TLR7, TLR8, or TLR9.

As used herein, the term "ubiquitin ligases" or "E3" are used interchangeably, refers to the third enzyme required for binding ubiquitin to target proteins, playing an important role in selectively recognizing and binding to specific target proteins and degrading target protein substrates mediated by ubiquitin. Based on different domains in the recognized target protein sequences, E3 can be divided into two major types: (1) ubiquitin ligases of the HECT domain family (HECT E3s), including Smurf2, E6, AP, ARF, BPI, etc.; (2) ubiquitin ligases of the RING domain family (RING E3s). The most typical characteristic of the RING domain family is having ring finger domains, which are important factors for the family to have the effects of ubiquitin ligases. RING E3s include a large number of members, such as: Mdm2 (Hdm2), APC, SCF, IAP, Skp2, etc.

The present invention discloses, for the first time, the negative regulatory effect of E3 on FOXP3. E3 used in the preferred embodiments of the present invention includes (but not limited to): STUB1, TRAF6, Smurf1, Smurf2, TRAF2, TRAF3, TRAF4, p300, or RBCK1, preferably STUB1. E3 used in the present invention preferably also has the molecular partner binding function or activity. STUB1 (STIP1 homology and U-Box containing protein 1) also has auxiliary molecular partner binding function and E3 ubiquitin-protein ligase activity. It is a molecular switch between the ubiquitin-proteasome system for abnormal protein degradation and the lysosomal system, and can coordinate the balance and stability of protein quality control system in cells, playing a key role in protein quality control.

As used herein, the "agonists" of ubiquitination pathway-related factors refers to materials capable of inducing the expression of ubiquitination pathway-related factors or increasing the activity of ubiquitination pathway-related factors, can also be called "inducers" in the present invention. Agonists used in the invention include, but not limited to: IL6.

As used herein, "antagonists" of the ubiquitination pathway-related factors refers to materials capable of inhibiting the expression of ubiquitination pathway-related factors or decreasing the activity of ubiquitination pathway-related factors, can also be called "inhibitors" in the present invention. Antagonists used in the invention include, but not limited to: MG-132.

The above proteins or polypeptides of the present invention can be purified natural products, or synthetic products, or generated by using recombinant technology from prokaryotic or eukaryotic host cells (for example, bacteria, yeast, plants, insects, and mammalian cells).

It should be understood, the above terms and definitions of the present invention also include conservative variant polypeptides of the proteins or polypeptides, or their homologous polypeptides. In one embodiment of the present invention, the proteins or polypeptides come from human and other eukaryotic organisms, such as mice, rats, cattle, or monkeys, etc., having high conservation among them.

Types of mutation of the protein or polypeptide variants of the present invention include (but not limited to): deletion, insertion, and/or substitution of one or more (usually 1-50, preferably 1-30, more preferably 1-20, most preferably 1-10, such as 1, 2, 3, 4, 5, 6, 7, 8, 9 or 10) amino acids, and addition of one or more (usually fewer than 20, preferably fewer than 10, more preferably fewer than 5) amino acids to C terminus and/or N terminus. For example, in the present technical field, the function of proteins or polypeptides usually does not change with substitution of amino acids having close or similar functions. Further, for instance, addition of one or more amino acid to C terminus and/or N terminus usually does not alter the functions of the proteins or polypeptides. One skilled in the art could easily confirm these mutation styles, which do not affect the activity of the proteins or polypeptides, according to common knowledge and/or conventional experiments.

In the present invention, "conservative polypeptide variants" refers to polypeptides, as compared with the known amino acid sequences of TLR or E3, have up to 20, preferably up to 10, more preferably up to 5, most preferably up to 3 amino acids substituted with amino acids with similar or close function, and still have identical or similar function of the original TLR or E3.

As used herein, the term "coding sequences" refers to sequences encoding the ubiquitin ligases or Toll like receptors described in the present invention, or their highly homologous sequences or molecules hybridized with the sequences under strict conditions, or gene family molecules highly homologous to the molecules described above.

As used herein, the term "stringent conditions" refers to: (1) hybridization and wash at relatively low ionic strength and relatively high temperature, such as the 0.2 X SSC, 0.1% SDS, 60°C; or (2) hybridization with denaturing agents, such as 50% (v/v) formamide, 0.1% calf serum/0.1% Ficoll, 42°C; or (3) hybridization takes place only when the identity between two sequences is at least 50%, preferably more than 55%, more than 60%, more than 65%, more than 70%, more than 75%, more than 80%, more than 85%, or more than 90%, and more preferably above 95%. For example, the sequences may be the complementary sequences to the sequences defined in (a).

The coding sequences for the full-length sequence of the present invention or their fragments can usually be obtained by using PCR amplification, recombinant or synthetic methods. For PCR amplification, primers can be designed according to the related nucleotide sequences disclosed by the present invention, especially, the open reading frame sequences; and use commercially available cDNA libraries or cDNA library prepared using conventional methods known to one skilled in the art as templates, and amplify to obtain the related sequences. When the sequences are longer, two or more PCR amplification are often required, and then splice together the amplified fragments according to the correct order.

### Compositions

The regulatory factors of the present invention can also be used for preparing therapeutic or preventive pharmaceutical compositions, healthcare compositions, or vaccine compositions.

Therefore, on the other hand, the invention also provides a composition, which contains (a) a safe and effective amount of the regulatory factors of the present invention; and (b) pharmaceutically acceptable carriers or excipient. The amount of the regulatory factors of the present invention is usually 10 µg -100 mg/agent, preferably of 100-1000 µg/agent.

As used herein, the term "effective amount" refers to the amount of therapeutic agent that treats, alleviates, or prevents the target diseases or conditions, or the amount that exhibits detectable treatment or prevention effect. Accurate effective amount for a given object depends on object size and state of health, nature and stage of illness, and the given therapeutic agents and/or combination of therapeutic agents. Therefore, pre-specified accurate effective amounts are useless. However, for a given condition, the effective amount can be determined using conventional experiments, clinicians should be able to decide.

Pharmaceutical compositions may also contain pharmaceutically acceptable carriers. The terms "a pharmaceutically acceptable carriers" refers to carriers used for delivery of therapeutic agents. This term refers to such medicament carriers: they, of themselves, do not induce the production of antibodies harmful to the individuals received the compositions, and there is no excessive toxicity after drug administration. These carriers are well known to one skilled in the art. A full discussion related to pharmaceutically acceptable excipients can be found in Remington's Pharmaceutical Sciences (Mack Pub. Co., N.J. 1991). This type of carriers includes (but not limited to): saline, buffer solution, glucose, water, glycerin, ethanol, adjuvant, and their combinations.

Pharmaceutically acceptable carriers in therapeutic compositions may contain liquid, such as water, saline, glycerol, and ethanol. In addition, these carriers may also have auxiliary materials, such as emulsifiers or wetting agents, pH buffer materials, etc. In addition, immune compositions may also contain immune adjuvant.

Usually, therapeutic compositions can be prepared as injectable agents, such as liquid solution or suspension; also can be prepared as pre-injection solid form suitable for blending into solutions or suspensions, and liquid carriers.

Once the compositions of the present invention are prepared, they can be given directly to subjects. The objects of prevention or treatment can be animals; especially, human.

Therapeutic or preventive pharmaceutical compositions (including vaccines) containing the regulating factors of the present invention can be applied through oral, subcutaneous, intradermal, and intravenous injection. Therapeutic dosage regimen can be single agent scheme or multiple agents scheme.

### Main advantages of the present invention

(1) the present invention discloses, for the first time, a use of ubiquitination pathway-related factors - - ubiquitin ligases, Toll like receptors, pro-inflammatory cytokine family receptors and/or their coding sequences as regulatory factors of FOXP3 activity, IL-2 activity, and/or IFN-γ activity;

(2) confirmation and application of the regulatory factors of the present invention provide new approaches for treating and/or preventing diseases or symptoms (e.g. tumor) associated with de-regulation of FOXP3, IL-2, and/or IFN-γ activity, by regulating FOXP3, IL-2, and/or IFN-γ activity to regulate regulatory T cells;

(3) the present invention also provides new methods of enhancing vaccine immunogenicity by using the regulatory factors of the present invention as vaccine adjuvant.

### Exampels

The following examples further elaborate the present invention. It should be understood, these exmples are used only to illustrate the present invention and not to limit the scope of the present invention. One skilled in the art can make suitable modifications and changes, these modifications and changes are all within the scope of the present invention.

Specific conditions of the experimental methods not indicated in the following embodiments can be used based on conventional methods the field, such as reference «Molecular Cloning» (Third Edition, New York: Cold Spring Harbor Laboratory Press, 1989) or according to the conditions recommended by the suppliers. DNA sequencing methods are conventional methods in the field, and can also be performed by commercial providers.

Unless otherwise specified, the percentage and copy number are calculated by weight. Unless otherwise defined, all the professional and scientific terminologies used herein have the same meaning as that familiar by one skilled in the art. In addition, any methods and materials similar to or equivalent to that disclosed herein can all be used in the methods of the present invention. Methods and materials of the preferred embodiments described herein are for demonstration purposes only.

### Materials and methods

### 1. Plasmids and vectors:

N terminus labeled HA-, Myc-, FLAG-FOXP3, eukaryotic expression vectors for various FOXP3 fragments; STUB1, Ub (ubiquitin) and pIPMyc2-STUB1, pIPHA2-FOXP3, other E3 plasmids (pIPHA2, pIPMyc2, pIPFLAG2 are eukaryotic expression vectors; pET28a and pET21-MBP are prokaryotic expression vectors) and lentiviral packaging plasmids (FUGW, de18.9, and VSV-G) were cloned and constructed by the present inventors' laboratory (see ref 4).

El, UbcH5b and HSP70 genes were obtained from human peripheral blood mononuclear cell cDNA library through PCR. Primers were designed according to the sequences downloaded from Genbank as follows:

| **Primer** | **SEQ ID NO:** | **Sequence** |
|---|---|---|
| E1-upstream primer | 1 | 5'-ATGTCCAGCTCGCCGCTGTCC-3' |
| E1-downstream primer | 2 | 5'-TCAGCGGATGGTGTATCGGAC-3' |
| UbcH5b-upstream primer | 3 | 5'-ATGGCTCTGAAGAGAATCCACAAG-3' |
| UbcH5b-downstream primer | 4 | 5'-TTACATCGCATACTTCTGAGTCCATTC-3' |
| HSP70-upstream primer | 5 | 5'-ATGGCCAAAGCCGCGGCGATC-3' |
| HSP70-downstream primer | 6 | 5’-TAATCTACCTCCTCAATGGTGGGGCCTG- 3’ |

TLR signaling downstream kinase IRAK1 expression plasmid, pIPIRAK1, was cloned and constructed by the present inventors' laboratory (see ref 4, the full gene sequence of IRAK1 referenced to Entrez Gene: 3654); ubiquitin ligase TRAF6 was provided by Professor Yongwon Choi (see ref 7, or obtained commercially (such as Origin company), or cloning by conventional methods).

### 2. Antibodies

Anti-HA antibody (F-7) and anti-Myc antibody (9E10) were purchased from Santa Cruz Company. Anti-Flag antibody (M2) was purchased from Sigma Company. Anti-FOXP3 antibody (hFOXY) was purchased from eBioscience Company. Anti-STUB1 antibody (C3B6) was purchased from Cell Signaling Company. Secondary anti-mouse antibody coupled with HRP was purchased from Promega Company.

### 3. Cells

Culture human HEK293T (purchased from Chinese Academy of Sciences Cell Bank (catalog number: GNHul7)) in DMEM (Dulbecco's modified Eagle's medium) containing 10% FBS, 100 units/ml penicillin, 37°C, 5% CO₂.

Culture Jurkat E6.1 T cells (purchased from the Chinese Academy of Sciences Cell Bank (catalog number: TCHU123)) in 1640 medium containing 10% FBS, 100 units/ml penicillin, 100 units/ml non-essential amino acids, and 100 units/ml sodium pyruvate, 37°C, 5% CO₂.

Cell transfection: use Lipofectamin 2000 (Invitrogen) for liposomal transfection. Samples were collected and analyzed at 48 hours post-transfection.

FUGW-TAP-FOXP3, del 8.9 and VSV-G were co-transfected into human embryonic kidney cell line HEK293. Supernatants of culture media were collected at 48 hours and 72 hours post-transfection. Infect Jurkat E6.1 with ultracentrifugation purified viruses to finally obtain TAP-FOXP3 Jurkat stable expression cell lines.

### 4. Reagents, nuclei, and separation of subcellular chromatin fractions:

Various TLR ligand reagents were purchased from Apotech Company.

MG132 was purchased from Merck Company.

Protein AG-beads were purchased from Shanghai Yue Ke Biological Technology Company.

After cells were lysed using cytoplasm extracting solution (10 µM Hepes, pH 7.9, 10 mM KCl, 0.1 mM EDTA, 1 mM DTT, 0.5 mM PMSF, 1 % complete protease inhibitor cocktails (Cat. No. 1-697-498; Roche Biochem), 1 µM Na₃VO₄), placed on ice for 15 minutes, then NP-40 was added to the final concentration of 0.6%, and vortexed for 30 seconds, centrifuged at 12000g for 30 seconds. The obtained supernatant is cytoplasmic fraction, and the precipitate is nuclear components. After nuclear fractions were dissolved using nuclear suspension solution (20 mM Hepes, pH 7.9, 400 mM NaCl, 1 mM EDTA, 1 mM DTT, 1 mM PMSF, 1' protease inhibitor cocktails, 1 mM Na₃VO₄), placed at 4°C with even speed slowly rotating for 30 minutes, and then centrifuged at 16,060 g, at 4°C for 15 minutes. The obtained insoluble precipitates are chromatin subfractions.

### 5. Immunoprecipitation

Lyse cells with RIPA buffer (20mM Tris/HCl, pH 7.5, 150mM NaCl, 1% NP-40, 0.5% Na-DOC, 1mM EDTA and 1mM PMSF protease inhibitors, 1 X Cocktail, phosphatase inhibitors 1mM Na₃VO₄, 1mM NaF). First add the primary antibody to cell lysate and incubate for 1 hour, and then add Protein A/G-coupled agarose beads and react for 1 hour. After washing three times by RIPA buffer, binding proteins were detected by SDS-PAGE.

### 6. Immunoblotting

After SDS-PAGE protein electrophoresis of protein samples, transfer to nitrate cellulose membranes, and then blocked in 5% skimmed milk powder TBST for one hour, add the primary antibody and incubate for one hour, and then add the secondary antibody coupled with HRP and incubate for one hour, and use ECL substrate for for chemiluminescence-based immunodetection.

### 7. Luciferase assay

The enhancer region of 8XFK luciferase reporter gene contains 8 repeats of Forkhead binding domains. The enhancer region of 5XGal luciferase reporter gene contains 5 repeats of Gal4 expression boxes. pBind-FOXP3 expression (see ref. 4) and Gal4 binding region fused with FOXP3. Transfect the corresponding plasmids into 293T cells, and use luciferase substrate and β-galactosidase substrate to measure the luciferase activity and the β-galactosidase activity. Each experiment was repeated independently more than three times. The obtained results are represented using mean values and standard deviations. The enzyme substrates were purchased from Beyotime Company.

### 8. Site-directed mutagenesis of genes

K30A and H260Q STUB 1 mutants were obtained through PCR (see ref. 5).

| **Primer** | **SEQ ID NO:** | **Sequence** |
|---|---|---|
| STUB1 K30A mutant primer 1 | 7 | GCGCGCAGGAGCTCGCCGAGCAGGGCAATCG |
| STUB1 K30A mutant primer 2 | 8 | CGATTGCCCTGCTCGGCGAGCTCCTGCGCGC |
| STUB1 H260Q mutant primer 1 | 9 | CATCGAGGAGCAACTGCAGCGTGTG |
| STUB1 H260Q mutant primer 2 | 10 | CACACGCTGCAGTTGCTCCTCGATG |

Site-directed mutagenesis kit was purchased from Stratagene Company, PCR followed by DNA sequencing verification.

### 9. Protein expression and purification

Transform Rosetta/PlysS competent cells with prokaryotic expression vectors carrying 6His-El, 6His-UbcH5b, 6His-STUB1, 6His-HSP70, and 6His-FLAG-Ubiqutin, induce expression by IPTG, collect cells, lyse cells, purify using nickel column chromatography (Qiagen Company), and, finally, obtain purified proteins using fast protein liquid chromatography (GE Company). Express MBP-FOXP3 and MBP proteins according to the above methods. Collect cells, break open cells, purify using amylose resin chromatography (NEB Company), use standard buffer solution to dialyze and elute proteins (25 mM Tris-HCl (pH 7.5), 150 mM N sodium chloride, 10 µM β-mercaptoethanol, and 10% glycerol). Finally, the Brandford method was used to determine all protein concentrations (Shanghai Beyotime Company).

### 10. in vitro ubiquitination assay

Add 0.1 µg 6His-El, 0.5 µg 6His-UbcH5b, 2 µg 6His-STUB1, 2 µg 6His-HSP70, 10 µg 6His-FLAG-Ubiquitin, and 2 µg MBP-FOXP3 or MBP to 100 µl reaction solution containing 50mM Tris/HCl (pH 7.4), 2mM magnesium chloride, 1mM DTT, and 4mM ATP. Mix evenly and incubate at 30°C for two hours, followed by adding SDS sample buffer to stop the reaction. Perform Western blotting, and use anti FLAG tag antibody to analyze the extent of ubiquitination in each system.

### 11. Immunofluorescence

Fix cell with 4% formaldehyde, perforate cell membrane with 0.5% Triton-X 100, blocking and label with the corresponding antibody. DAPI labels nuclei. Finally, place cells under confocal microscope to observe results (LEICA SP5).

### 12. Cell proliferation assay

Isolate lymphocytes from Thyl.2 mice (purchased from Jackson laboratories of the United States) and Thyl. 1 mouse (purchased from Jackson laboratories of the United States). Obtain regulatory T cells and CD4⁺CD25⁻CD62L^{high} effector T cells through antibody labeling and magnetic bead selection. As described before, after labeling effector T cells using 1 µM CFSE (Invitrogen Company), culture the regulatory T cells and effector T cells for 80 hours, and detect fluorescence intensity changes in effector T cells and using flow cytometry to analyze cell proliferation.

### 13. Construction of colitis model and immunohistochemistry

Isolate lymphocytes from BALB/c mice (purchased from Jackson laboratories of the United States). Obtain natural CD4⁺CD25⁻CD62L^{high} effector T cells through antibody labeling and magnetic bead selection, and inject into BALB/c RAG2-/- immunodeficient mice (purchased from Taconic Company of the United States) via tail veins (∼1 x 10⁶ cells/mouse). The experimental mice were divided into two groups, inject, respectively, through tail veins with wild-type CD4⁺CD25⁺Treg cells and STUB1-transfected Treg cells (2 x 10⁵). Symptoms are monitored and assessed weekly. Experimental mice were euthanized once the symptoms became severe or had 20% weight losses.

8 weeks after T cell reconstitution, colonic tissues were removed from mice, fixed in 10% formaldehyde, embedded and sectioned in paraffin, and stained with hematoxylin-eosin. Assessment of colonic tissue lesions, grade 0: without obvious lesion; grade 1: low degree of immune cell infiltration, with or without accompanied minor endothelial cell proliferation; grade 2: mild immune cell infiltration, accompanied with mild to moderate endothelial cell proliferation and minor to moderate reduction of secreted mucus from rod-shaped cells; grade 3: moderate immune cell infiltration, moderate to severe endothelial cell proliferation and moderate to severe reduction of secreted mucus from rod-shaped cells; grade 4: high degree of immune cell infiltration occurring in the bowel wall, high endothelial cell proliferation and severe reduction of secreted mucus; grade 5: high immune cell infiltration in the bowel wall accompanied with the symptoms of ulcerative inflammation, and loss of tubular intestinal glands.

### Example 1. Effect of TLR signaling on FOXP3

*Test 1. Effect of TLR signaling on expression levels of FOXP3*

Jurkat T cells were transfected with FOXP3 expression plasmid Myc-FOXP3a , 48 hours later, stimulated by adding different TLR ligands (operated according to the procedure of Apotech kit). After cell samples were collected, nuclei and chromatin factions were isolated. Detection was performed, respectively, by protein electrophoresis and immunoblotting.

The experimental results as shown in FIG. 3a. The results show that the FOXP3 protein expression changes with different TLR signaling stimulations. FOXP3 protein levels were significantly reduced by the effect of TLR signaling (especially, TLR3 and TLR9).

These results show that: TLR signaling (such as, TLR3 and TLR9) can serve as negative regulatory signaling of FOXP3.

*Test 2. Effect of TLR signaling downstream activator protein TRAF6 on FOXP3 ubiquitination*

293T cells were co-transfected with TLR signaling downstream activator protein TRAF6 expression plasmid and FOXP3 expression plasmid Myc-FOXP3a. Cells were collected 48 hours later. Immunoprecipitation was performed and, then, detection, respectively, by protein electrophoresis and immunoblotting.

The experimental results as shown in FIG. 3b. The results show: co-expression of TLR signaling downstream adaptor protein TRAF6 having ubiquitin ligase activity can significantly promote FOXP3 ubiquitination.

The results show that: TLR signaling (such as, TLR3 and TLR9) can promote FOXP3 ubiquitination through its downstream ubiquitin ligase TRAF6.

*Test 3. Effect of TLR signaling downstream activator protein IRAK1 on FOXP3 ubiquitination*

293T cells were co-transfected with TLR signaling downstream kinase expression plasmid IRAK1 and FOXP3 expression plasmid Myc-FOXP3a. Cells were collected 48 hours later. Immunoprecipitation was performed and, then, detection, respectively, by protein electrophoresis and immunoblotting.

The experimental results as shown in FIG. 3c. The results show: co-expression of TLR signaling downstream kinase expression plasmid IRAK1 can significantly promote FOXP3 ubiquitination.

The results show that: TLR signaling (such as, TLR3 ,TLR4 and TLR9) can promote FOXP3 ubiquitination through its downstream kinase IRAK1.

### Example 2. FOXP3 binding to STUB1 and other E3

293T cells were co-transfected with HA-FOXP3a and Myc-STUB1, and co-immunoprecipitated, respectively, using anti-HA and anti-Myc antibodies, and detection by immunoblotting (results shown in FIG. 4(A), left panel). In reversed experiments, 293T cells were co-transfected with Flag-FOXP3 and Myc-STUB1, co-immunoprecipitated, respectively, using anti-Flag and anti-Myc antibodies, and detection by immunoblotting (results shown in FIG. 4(A), right panel).

Other immune signaling pathways can be detected by co-transfecting 293T cells with E3 and Flag-FOXP3a, HA-Ubi. Cell lysate was immunoprecipitated with anti-Flag antibody, and detection with anti-Myc antibody (results as shown in FIG. 4(B)).

The results show: FOXP3 can be co-immunoprecipitated with STUB1, and STUB1 can also specifically pull down FOXP3, suggesting that the interaction between STUB1 and FOXP3 is specific and not dependent on the expression of exogenous labels. In addition, ubiquitin ligases, which play key role in other signaling pathways, can also interact with FOXP3.

The results show: FOXP3 protein can interact with ubiquitin ligase, and modified by ubiquitination. In particular, FOXP3 protein can interact with STUB1 ubiquitin ligase regulated by stress signaling.

### Example 3. STUB1 can induce FOXP3 ubiquitination

293T cells were co-transfected with Myc-STUB1, HA-Ub, and Flag-FOXP3a or Flag-FOXP1. Cell lysate was immunoprecipitated with anti-HA antibody, and detection of ubiquitinated protein by immunoblotting (results as shown in FIG. 5 (A)). Jurkat T cells were co-transfected with Myc-STUB1, HA-Ub, and Flag-FOXP3a. Cell lysate was immunoprecipitated with anti-HA antibody, and detection by immunoblotting (results as shown in FIG. 5 (B)).

The results show: ubiquitin ligase STUB1 can specifically act on FOXP3, and induce ubiquitination of FOXP3 protein.

The results show: ubiquitination is one of post-translational modifications of FOXP3 and FOXP3 is a specific substrate of STUB1 ubiquitin ligase.

### Example 4. STUB 1 promotes FOXP3 degradation by ubiquitination

293T cells were co-transfected with Flag-FOXP3, HA-Ub, and Myc-STUB1. Prior to cell collection, treatment with 5 µM MG132 at different times. Lysate was immunoprecipitated with anti-Myc antibody and detection by immunoblotting (results as shown in FIG. 6 (A)).

293T cells were co-transfected with Flag-FOXP3a, HA-Ub, and Myc-STUB1 or STUB1-K30A mutant deficient of binding to Chaperone or STUB1-H260Q mutants deficient of ubiquitin ligase activity. After cells were treated with 5 µM MG132 treatment for 4 hours, immunoprecipitated with anti-HA antibody and detection by immunoblotting (results as shown in FIG. 6 (B)).

The results show: MG132 inhibition can significantly enhance the ubiquitinated FOXP3 protein levels. STUB1 capable of binding to molecular Chaperone and having ubiquitin ligase activity can induce FOXP3 degradation by ubiquitination. In addition, although ubiquitin ligase activity-deficient mutant H260Q-STUB1 can still bind FOXP3, both H260Q and K30A STUB1 mutants can not promote FOXP3 ubiquitination, suggesting that the enzymatic activity of STUB1 has an important effect on ubiquitination of FOXP3.

The results show: STUB1 negatively regulates FOXP3 protein levels by inducing FOXP3 degradation by ubiquitination.

### Example 5. STUB1 inhibits FOXP3 transcription activity

8 X FK luciferase reporter system was used to detection FOXP3 activity. 293T cells were co-transfected with FOXP3, STUB1, 8 X FK luciferase reporter gene and MSV-β-Gal control. After the luciferase activity was normolized using β-Gal activity, results obtained from three independent experiments were presented using mean values ± standard deviations (results as shown in FIG. 7 (A)).

293T cells were co-transfected with pBind-FOXP3, STUB1, and pGal5-luciferase reporter gene and MSV-β-Gal control using pBind system (constructed according to ref 4). After the luciferase activity was standardized using β-Gal activity, results obtained from three independent experiments were presented using mean values ± standard deviations (results as shown in FIG. 7 (B)).

The results show: FOXP3, as a transcriptional repressor, can reduce the transcriptional activity of the reporter gene, whereas addition of STUB1 weakens the transcription inhibitory ability of FOXP3.

The results show: STUB1, as a negative regulatory factor of FOXP3, can inhibit FOXP3 function.

The present inventors further tested the effect of HSP70 and STUB1 overexpression on the transcription repression of luciferase reporter gene, driven by FOXP3-mediated IL-2 promoter, expression levels. Jurkat T cells were transfected with corresponding plasmids. Prior to sample collection, the transfected cells were stimulated with PMA and ionomycin, followed by analysis of luciferase activity expressed in cells. We found, FOXP3 significantly inhibited luciferase expression levels driven by IL-2 promoter and no effect of HSP70 expression on the inhibition. However, co-expression of FOXP3 and STUB1 almost completely reversed the inhibitory effect of FOXP3 on IL-2 promoter. Also, HSP70 can significantly enhance the reversal effect of STUB1 on FOXP3 transcription repression (FIG. 7 (C)).

The previously reported research results showed, FOXP3 has an inhibitory effect on IL-2 expression in primary T cells. Therefore, we used lentivirus-mediated transfection methods to express full-length FOXP3 protein and STUB1 in primary CD4⁺T cells to study whether or not STUB1 can alleviate the inhibitory effect of FOXP3 on IL-2 expression. Consistent with that in Jukart cells, FOXP3 almost completely inhibited IL-2 expression in naive T cells. However, STUB1 overexpression can reverse the inhibitory effect of FOXP3 on IL2 expression (FIG. 7 (D)).

### Example 6. Identification of FOXP3 critical regions interacting with STUB1, effects of molecular binding partners in interactions, and possible mechanisms

The present inventors also identified FOXP3 critical regions interacting with STUB1. As shown in FIG. 8 (A), the zinc finger and leucine zipper domains of FOXP3 are proven to be the essential regions binding to STUB 1.

To test whether or not the interaction between FOXP3 and STUB1 is dependent on molecular binding partners HSC70/HSP70, 293T cells were co-transfected with HA-FOXP3 and Myc-labeled wild type STUB1, or STUB1-K30A mutant deficient of molecular partner binding or STUB1-H260Q mutant deficient of ubiquitin ligase activity, followed by co-immunoprecipitation of STUB1 with anti-Myc antibody. Subsequently, anti-HA antibody and anti-Myc antibody were used for immunoblotting detection. The results show, both wild type STUB1 and H260Q-STUB1 mutant can bind to FOXP3, but K30A-STUB1 mutant deficient of molecular partner binding cannot bind to FOXP3 (FIG. 8 (B)).

To verify whether or not STUB 1 can induce down-regulation of FOXP3 expression levels, co-expressed HA-FOXP3, Myc-STUB1, FLAG-HSP70 in 293T cells, and cells without FLAG-HSP70 transfection served as control. The results show: STUB1 and HSP70 can synergistically promote FOXP3 degradation (FIG. 8 (C)).

These results suggested that HSC70 and HSP70 molecular binding partners are required mediator proteins for the interaction of FOXP3 and STUB 1.

Immunofluorescence analysis shows, STUB1 can be expressed in large amount in the peripheral Jurkat cells stably expressing TAP-FOXP3. We also found, under heat shock stimulus conditions, STUB1 can gradually aggregate inside nucleus (FIG. 8 (D)). Changes of STUB1 localization under heat shock conditions reflect increased opportunities of contacting with FOXP3, thus, promoting their binding. This process may be a mechanism of FOXP3 degradation under stress conditions, thereby regulating immunity.

### Example 7. STUB1 negatively regulates of FOXP3 protein expression and mouse primary Treg cell function in vitro

To test whether or not STUB1 can negatively regulate FOXP3 expression in mouse primary Treg cells *in vitro,* the following experiments were performed: primary Treg cells were isolated from C57BL/6 mice, and infected with lentiviral empty vector (carrying GFP tag) or carrying STUB1 gene. As shown in FIG. 9 (A), as compared to the empty vector control group, STUB1 overexpression decreases FOXP3 expression in cells, while increases inflammatory factor IL-2 and IFN-γ expression (FIG. 9 (B) and 9 (C)). The above results suggest that Treg cells partially lose gene silencing function.

In addition, the present inventors further confirmed that STUB1 overexpression can greatly affect the function of Treg cells to inhibit cell proliferation through cell proliferation experiments (FIG. 9 (D)).

These research findings suggest that expressing STUB1 in primary Treg cells can reduce the expression levels of FOXP3, and affect its function.

### Example 8. STUB1 overexpression in Treg cells in vivo can reduce immune suppression function of Treg cells in experimental colitis mouse model

To test whether or not STUB1 overexpression can affect Treg cell function *in vivo,* natural CD4⁺CD25⁻CD62L^{high} T cells were transplanted into Rag2^{-/-} mice, constructed Thl-mediated mouse colitis model. In this model, the co-transplanted CD4⁺CD25⁺Treg cells can effectively inhibit inflammation, and control disease development.

In this experiment, the colitis development was determined by monitoring mouse body weight and immunohistochemistry analysis. As shown in FIG. 10 (A), compared with the control group, the body weight of mice transplanted with Teff (effector T cells) and STUB1-overexpressing Treg cells significantly decreases, the degree of inflammation shown in immunohistochemistry slides significantly increases, and cannot effectively inhibit inflammation development (FIG. 10 (B) and 10 (C)).

The above *in vivo* experimental results are consistent with that of the *in vitro* experiments, and, once again, proved that STUB1 is a negative regulatory factor of FOXP3 and also a key regulation factor of Treg cell function.

### Example 9. Ubiquitination pathways related factors IL-6 and TGF-β promote FOXP3 degradation

CD4⁺T cells, under different cytokines and environmental factors, can be differentiated into four different subtypes: TH1, TH2, TH17, and iTreg. These four T cell subtypes have their specific cytokines and transcription factors. The previous studies found, under the effect of TGF-β and inflammatory factor IL-6, CD4⁺T cells differentiate into TH17, whereas, under the effect of TGF-β, CD4⁺T cells can differentiate into iTreg. TGF-β plays a promoting role in TH17 differentiation and IL-6 plays an inhibitory role in iTreg differentiation, their mechanisms remain unclear.

This example proves the IL-6 and TGF-β synergism can promote FOXP3 degradation through Western blotting and FACS, etc., primarily considering the mechanisms of degradation in an ubiquitination-dependent manner.

Cell lines used in Western blotting and FACS experiments were stably transfected Jurkat-HA-FOXP3 (as described in Materials and Methods); the amount of IL-6 added was 20ng/ml; and the amount of TGF-β added was 10ng/ml. Staining by using IL-6R antibody (purchased from Santa Cruz Company), and Cy3-labeled anti-Rabbit secondary antibody (purchased from Jackson Company), detection was performed through flow cytometry PE channel. Results of Western blotting are shown in FIG. 11, and results of FACS as shown in FIGs. 12 and 13.

As can be seen form the Western blotting results shown in FIG. 11, when IL-6 acts alone, FOXP3 expression levels do not change, while, under the effect of IL-6/TGF-β, FOXP3 expression levels began to decline at 12 hours.

As can be seen form FACS results shown in FIG. 12, TGF-β promotes the expression of IL-6 receptors, but IL-6 has no effect on the expression of its own receptor. It has been reported that TGF-β can inhibit SOCS3, the negative feedback factor of TH17, thereby can interact with TH17 specific cytokines, IL-6 and IL-21, promoting TH17 differentiation. Therefore, TGF-β promotes TH17 differentiation through a variety of mechanisms, for example: in the presence of IL-6, promotes FOXP3 degradation, and up regulates IL-6 receptors, etc.

Further, as can be seen from FACS results shown in FIG. 13, TGF-β can significantly promote FOXP3 expression. However, under the effect of IL-6/TGF-β, FOXP3 expression levels began to decline at 12 hours, which are consistent with the up regulation of IL-6 receptors began at 12 hours described above. Thus, IL-6 can reverse the effect of TGF-β-promoted FOXP3 expression, and thereby promotes FOXP3 degradation through certain mechanisms.

All documents mentioned in the present invention are cited as references in the present application, similar to each document was separately cited as a reference. In addition, it should be understood, by reading the teaching of the present invention, one skilled in the art can change or modify the present invention. However, the equivalent forms also fall within the scope limited by the attached claims.

### References

1. Belkaid, Y" Piccirillo, C.A., Mendez, S" Shevach, E.M., and Sacks, D.L. (2002). CD4+CD25+ regulatory T cells control Leishmania major persistence and immunity. Nature 420, 502-507.
2. Belkaid, Y. and Rouse, B.T. (2005). Natural regulatory T cells in infectious disease. Nat Immunol 6, 353-360.
3. Li, B. and Greene, M.I. (2008). Special regulatory T-cell review: FOXP3 biochemistry in regulatory T cells-how diverse signals regulate suppression. Immunology 123, 17-19.
4. Li, B., Samanta, A., Song, X., Iacono, K.T., Bembas, K., Tao, R., Basu, S., Riley, J.L., Hancock, W W., Shen, Y., et al. (2007). FOXP3 interactions with histone acetyltransferase and class II histone deacetylases are required for repression. Proc Natl Acad Sci U S A 104, 4571-4576.
5. Qian , S. , McDonough , H., Boellman, F., Cyr, D M., Patterson, C, et al. (2006). CHIP-mediated stress recovery by sequential ubiquitination of substrates and Hsp70. Nature 440,551-555.
6. WO 2007/084775 , Composition and method for modulation of suppressor T cell activation.
7. Kobayashi T, Walsh PT, Walsh MC, Speirs KM, Chiffoleau E, King CG, Hancock WW, Caamano JH, Hunter CA, Scott P, Turka LA, Choi Y. TRAF6 is a critical factor for dendritic cell maturation and development. Immunity. 2003 Sep; 19(3):353-63.

## Claims

1. Use of a ubiquitination pathway-related factor, an agonist thereof, or an antagonist thereof in the preparation of a composition for regulation of FOXP3 activity, IL-2 activity, and/or IFN-γ activity, wherein the ubiquitination pathway-related factor is selected from: Toll like receptor, ubiquitin ligase, pro-inflammatory cytokine receptor, and/or their coding sequences.

2. The use of claim 1, **characterized in that** the regulation of FOXP3 activity, IL-2 activity, and/or IFN-γ activity is positive or negative regulation, wherein the ubiquitination pathway-related factor or the agonist is used for negative regulation of FOXP3 activity and positive regulation of IL-2 activity and/or IFN-γ activity, the ubiquitination pathway-related factor antagonist is for positive regulation of FOXP3 activity and negative regulation of IL-2 activity and/or IFN-γ activity.

3. The use of claim 1, **characterized in that** the pro-inflammatory cytokine receptor is selected from: IL-6R, TGF-β receptor, IL-2 receptor, TNF-α receptor, or GITR.

4. The use of claim 1, **characterized in that** the use is using the ubiquitination pathway-related factor in the preparation of a composition for the negative regulation of FOXP3 activity, wherein the ubiquitination pathway-related factor is selected from: Toll like receptor, ubiquitin ligase, and/or their coding sequences.

5. The use of any one of claims 1-4, **characterized in that** the ubiquitin ligase is one or more selected from: STUB1, TRAF6, Smurf1, Smurf2, TRAF2, TRAF3, TRAF4, p300, RNF31, or RBCK1.

6. The use of any one of claims 1-4, **characterized in that** the Toll like receptor is one or more selected from the group consisting of: TLR1, TLR2, TLR3, TLR4, TLR5, TLR7, TLR8, and TLR9.

7. The use of any one of claims 1-4, **characterized in that** the composition for the regulation of FOXP3 activity, IL-2 activity, and/or IFN-γ activity is used for treating and/or preventing a disease or a symptom associated with a de-regulation of FOXP3, IL-2, and/or IFN-γ activity.

8. The use of claim 7, **characterized in that** the de-regulation of activity is excessive high activity or excessive low activity.

9. The use of claim 7, **characterized in that** the disease or the symptom associated with the de-regulation of FOXP3, IL-2, and/or IFN-γ activity is selected from: tumor, inflammation, an acute infectious disease, or a chronic infectious disease.

10. The use of claim 9, **characterized in that**
the tumor is selected from: prostate cancer, breast cancer, liver cancer, glioma, colorectal cancer, cervical cancer, non-small cell lung cancer, lung cancer, pancreatic cancer, gastric cancer, bladder cancer, skin cancer, carcinoma, tongue squamous cell carcinoma, nasopharyngeal carcinoma, ovarian cancer, placental villous cancer, glioma, lymphoma, leukemia, rectal adenocarcinoma, or melanoma;
the inflammation is selected from: allergic inflammation, folliculitis, tonsillitis, pneumonia, hepatitis, nephritis, acne, asthma, autoimmune diseases, chronic inflammation, chronic prostatitis, glomerulonephritis, hypersensitivity, inflammatory bowel disease, pelvic inflammatory disease, reperfusion injury, rheumatoid arthritis, vasculitis, transplant rejection, or interstitial bladder inflammation; and
the infectious disease is selected from: plague, cholera, SARS, AIDS, viral hepatitis, polio, human infection of highly pathogenic avian influenza, measles, epidemic hemorrhagic fever, rabies, Japanese encephalitis, dengue fever, arthritis, hand-foot and mouth disease, anthrax, bacillary and amebic dysentery, tuberculosis, typhoid fever and paratyphoid fever, epidemic cerebrospinal meningitis, pertussis, diphtheria, tetanus, scarlet fever, brucellosis, gonorrhea, syphilis, leptospirosis, schistosomiasis, malaria, influenza, mumps, rubella, acute hemorrhagic conjunctivitis panniculitis, leprosy, epidemic and endemic typhus, black fever, echinococcosis, filariasis, an infectious diarrhea diseases other than cholera, bacillary and amebic dysentery, typhoid and paratyphoid fever, or a fungal infection.

11. The use of claim 1, **characterized in that** the composition for the regulation of FOXP3 activity, IL-2 activity, and/or IFN-γ activity is used as a vaccine adjuvant or a vaccine.

12. The use of claim 11, **characterized in that** the vaccine is selected from: a vaccine for treating or preventing viral infection, bacterial infection, inflammation, parasite infection, or tumor.

13. The use of claim 11, **characterized in that** the vaccine is hand-foot-and-mouth disease vaccine.

14. The use of claim 1, **characterized in that** the composition is a pharmaceutical composition, a healthcare composition, or a vaccine composition.

15. A composition for regulating an activity of FOXP3, IL-2, and/or IFN-γ, comprising:
(a) one or more ubiquitination pathway-related factors selected from the group consisting of: ubiquitin ligase, Toll like receptor, pro-inflammatory cytokine family receptor and/or their coding sequences; and/or an agonist or an antagonist of the ubiquitination pathway-related factor; and
(b) a pharmaceutically, immunologically, or healthcare acceptable carrier.

16. The composition of claim 15, **characterized in that** the ubiquitin ligase is one or more selected from the group consisting of: STUB1, TRAF6, Smurf1, Smurf2, TRAF2, TRAF3, TRAF4, p300, RNF31 and RBCK1; the Toll receptor is one or more selected from the group consisting of: TLR1, TLR2, TLR3, TLR4, TLR5, TLR7, TLR8, and TLR9; and the pro-inflammatory cytokine receptor is selected from the group consisting of: IL-6R, TGF-β receptor, IL-2 receptor, TNF-α receptor, and GITR.


SEQUENCE LISTING

<110>  LI, Bin
       LIN, Fang
       CHEN, Zuojia
       LI, Zhiyuan
       PAN, Fan

<120>  REGULATORY FACTOR OF FOXP3 AND REGULATORY T CELLS AND USE THEREOF

<130>  17257/023001

<150>  PCT/CN2011/077032
<151>  2011-07-11

<150>  CN 201010222995.8
<151>  2010-07-09

<160>  10

<170>  PatentIn version 3.3

<210>  1
<211>  21
<212>  DNA
<213>  Artificial Sequence

<220>
<223>  Primer

<400>  1
atgtccagct cgccgctgtc c                                                21


<210>  2
<211>  21
<212>  DNA
<213>  Artificial Sequence

<220>
<223>  Primer

<400>  2
tcagcggatg gtgtatcgga c                                                21


<210>  3
<211>  24
<212>  DNA
<213>  Artificial Sequence

<220>
<223>  Primer

<400>  3
atggctctga agagaatcca caag                                             24


<210>  4
<211>  27
<212>  DNA
<213>  Artificial Sequence

<220>
<223>  Primer

<400>  4
ttacatcgca tacttctgag tccattc                                          27


<210>  5
<211>  21
<212>  DNA
<213>  Artificial Sequence

<220>
<223>  Primer

<400>  5
atggccaaag ccgcggcgat c                                                21


<210>  6
<211>  28
<212>  DNA
<213>  Artificial Sequence

<220>
<223>  Primer

<400>  6
taatctacct cctcaatggt ggggcctg                                         28


<210>  7
<211>  31
<212>  DNA
<213>  Artificial Sequence

<220>
<223>  Primer

<400>  7
gcgcgcagga gctcgccgag cagggcaatc g                                     31


<210>  8
<211>  31
<212>  DNA
<213>  Artificial Sequence

<220>
<223>  Primer

<400>  8
cgattgccct gctcggcgag ctcctgcgcg c                                     31


<210>  9
<211>  25
<212>  DNA
<213>  Artificial Sequence

<220>
<223>  Primer

<400>  9
catcgaggag caactgcagc gtgtg                                              25

<210>  10
<211>  25
<212>  DNA
<213>  Artificial Sequence

<220>
<223>  Primer

<400>  10
cacacgctgc agttgctcct cgatg                                              25